# EUROPEAN PATENT APPLICATION

(11) **EP 4 748 293 A1**
(43) Date of publication of application: **27.05.2026**
(21) Application number: 24855087.3
(22) Date of filing: 30.09.2024
(51) Int. Cl.: A61B 1/012, A61B 1/00

(54) **CONNECTING APPARATUS AND ENDOSCOPE CONNECTING SYSTEM**

(71) Applicant: Jiangsu Vedkang Medical Science and Technology Co., Ltd., Changzhou, Jiangsu 213149 (CN)
(72) Inventor: DING, Qiushi, Changzhou, Jiangsu 213149 (CN); LU, Hualing, Changzhou, Jiangsu 213149 (CN)
(74) Representative: karo IP
(86) International application number: PCT/CN2024/122899
(87) International publication number: WO 2026/065451

(57) **Abstract**

Embodiments of the present application provide a connecting device and an endoscope connecting system. The connecting device includes a first joint portion and a second joint portion. The first joint portion is used for connecting a child endoscope, and the second joint portion is connected to the first joint portion. The second joint portion encloses to form a sleeving cavity and a first opening and a second opening in communication with the sleeving cavity, a cross-sectional area of the sleeving cavity gradually increases in a direction from the first opening to the second opening, and the second joint portion is used for being sleeved on a parent endoscope through the sleeving cavity, allowing the second joint portion to be connected to the parent endoscope. The second joint portion of the connecting device provided by the embodiments of the present application can be sleeved on the parent endoscope, so as to implement quick matching between the connecting device and the parent endoscope, and when the parent endoscope needs to be disassembled, it is only required that the parent endoscope moves upwards relative to the connecting device, the matching between the parent endoscope and the connecting device can be released. The connecting device is simple in structure, and the matching between the child endoscope and the parent endoscope are conveniently implemented and released.

## Description

### Technical Field

The present application relates to the technical field of medical instruments, and in particular, to a connecting device and an endoscope connecting system.

### Background Technology

Endoscopes are differentiated according to usage scenarios and can be classified into bronchoscopes, enteroscopes, and gastroscopes, etc. The endoscope is simply an examination instrument and usually includes two parts: a handle and an imaging catheter connected to the handle. The imaging catheter (with a camera arranged at the front end) is configured to perform photographing or video recording after entering a natural orifice of a human body. Images captured by the camera are transmitted to a host display connected to the handle of the endoscope through photoelectric transmission, so that a doctor can observe whether there is diseased tissue (such as polyp or early cancer) in the natural orifice of the human body. If the doctor discovers the diseased tissue by means of the camera of the imaging catheter, since the endoscope itself does not have a surgical function, the imaging catheter is usually provided with an instrument orifice, the handle is provided with a forceps channel hole, and the forceps channel hole is in communication with the instrument orifice. When diseased tissue, such as a polyp, needs to be resected, an endoscopic minimally invasive surgical consumable instrument, such as a snare, needs to be inserted from the forceps channel hole to reach the target diseased tissue via the imaging catheter, and then a polyp removal surgery is performed. After the resection surgery is finished, the snare is withdrawn from the handle of the endoscope. If the camera lens of the imaging catheter detects bleeding after the resection surgery, a hemostatic clip needs to be extended through the forceps channel hole, and the hemostatic clip is released to complete tissue clipping, thereby implementing hemostasis by compression.

Choledochoscope, as the name implies, is a tool for diagnosing and treating diseases (usually gallstones) that occur in the biliary tract. The biliary tract is elongated and narrow, while a diameter of an imaging catheter of a common endoscope, such as a duodenoscope, is close to 2 cm, which is obviously unsuitable for directly treating the diseased tissue of the biliary tract. The choledochoscope (which also includes a handle and an imaging catheter, the handle is also provided with a forceps channel hole, and the imaging catheter is also provided with an instrument orifice) serves as a minimally invasive surgical consumable instrument like the snare and the hemostatic clip described above. The imaging catheter of the choledochoscope is inserted into the forceps channel hole of the endoscope (generally a duodenoscope), and then further enters the biliary tract (the diameter of the imaging catheter of the choledochoscope being generally within 3 mm) via the imaging catheter of the duodenoscope, so as to perform a calculus removal or in vivo sampling operation.

It can be seen from the above that the choledochoscope, as a child endoscope of a duodenoscope (also referred to as a parent endoscope), is used together with the duodenoscope rather than independently. During surgery, the doctor needs to hold the duodenoscope and the choledochoscope at the same time, and the parent endoscope (the duodenoscope) cannot be moved freely after reaching the vicinity of the biliary tract, and therefore, it is required to keep relative fixation between the child endoscope (the choledochoscope) and the parent endoscope (the duodenoscope) during the surgery.

### Summary of the Invention

In order to solve one of the above technical defects, embodiments of the present application provide a connecting device and an endoscope connecting system, so as to solve a problem that a doctor needs to hold two medical instruments respectively during surgery and the operation of the surgery is relatively troublesome.

The present invention adopts the technical solutions as follows:
A first purpose of the present application is to provide a connecting device, including:
a first joint portion used for connecting a child endoscope; and
a second joint portion connected to the first joint portion, where the second joint portion encloses to form a sleeving cavity and a first opening and a second opening in communication with the sleeving cavity, a cross-sectional area of the sleeving cavity gradually increases in a direction from the first opening to the second opening, and the second joint portion is used for being sleeved on a parent endoscope through the sleeving cavity, allowing the second joint portion to be connected to the parent endoscope.

Optionally, the second joint portion is arranged in any one of the following manners:
the second joint portion is in a closed ring shape, and an outer wall of the second joint portion is connected to the first joint portion; and
the second joint portion is provided with a first notch, the first notch is in communication with the sleeving cavity, the first notch extends from a first port to a second port, and one side, opposite to the first notch, of the second joint portion is connected to the first joint portion.

Optionally, the first notch is a long strip-shaped opening; and
a width of the first notch is not less than an outer diameter of a local structure of the parent endoscope, so that the parent endoscope can extend into the sleeving cavity through the long strip-shaped opening.

Optionally, the connecting device includes a sheath, where the sheath is a flexible body;
the sheath is at least partially located in the sleeving cavity; and
in a state in which the second joint portion is connected to the parent endoscope, the sheath is disposed between the parent endoscope and an inner wall of the sleeving cavity in a compressed manner.

Optionally, the sheath is provided with a sheath main body and a port edge covering rib;
the sheath main body is provided with two ports, and the two ports are provided with the port edge covering rib, respectively; and
in a state in which the sheath is connected to the second joint portion, the sheath main body is attached to the inner wall of the sleeving cavity, and the two port edge covering ribs are covered on an end surface of an edge of the first opening of the second joint portion and an end surface of an edge of the second opening of the second joint portion, respectively.

Optionally, a second notch is arranged on the sheath main body, and the second notch is in communication with the two ports, respectively;
the sheath is provided with a notch edge covering rib arranged on the sheath main body, and the notch edge covering rib is located at an edge of the second notch; and
in the state in which the sheath is connected to the second joint portion, the notch edge covering rib is covered on a thickness end surface of an edge of the first notch of the second joint portion.

Optionally, the first joint portion includes a main housing and a plurality of clamping pieces;
the second joint portion is connected to the main housing;
each clamping piece is connected to the main housing, and each clamping piece is successively arranged at intervals along a periphery of an edge of the main housing; and in a state in which the first joint portion is connected to the child endoscope, the main housing is attached to the child endoscope, and each clamping piece is clamped to an outer wall surface of a medical instrument, respectively.

Optionally, a reinforcing rib is arranged on a surface of the main housing away from the second joint portion.

Optionally, a reinforcing back plate is arranged on a surface of the main housing away from each clamping piece; and
the second joint portion is connected to the reinforcing back plate.

Optionally, the child endoscope is provided with a plurality of functional portions; and
in a state in which the first joint portion is connected to the child endoscope, at least part of the functional portions are located on a gap formed between adjacent clamping pieces.

Optionally, the child endoscope is provided with a forceps channel nozzle protruding outwards, and the forceps channel nozzle is provided with a forceps channel opening; and
in the state in which the first joint portion is connected to the child endoscope, one clamping piece is clamped to an outer wall surface of the forceps channel nozzle.

Optionally, two sides of the first joint portion in a width direction are provided with a first side edge and a second side edge, respectively; and
a distance between the second joint portion and the first side edge is less than a distance between the second joint portion and the second side edge.

A second purpose of the present application is to provide an endoscope connecting system, including:
the above connecting device; and
a child endoscope detachably connected to a first joint portion of the connecting device.

Optionally, the endoscope connecting system further includes:
a parent endoscope detachably connected to a second joint portion.

Optionally, a disassembly direction of the child endoscope and the first joint portion is different from a disassembly direction of the parent endoscope and the second joint portion.

Optionally, an acting force is applied in a horizontal direction of an operating handle of the child endoscope, allowing that the child endoscope is split from the first joint portion.

Optionally, an acting force is applied in a vertical direction of an imaging catheter of the parent endoscope, allowing that the parent endoscope is split from the second joint portion.

By adopting the above technical solutions, the present application has the following beneficial effects:
the second joint portion of the connecting device provided by the embodiments of the present application can be sleeved on the parent endoscope, so as to implement quick matching between the connecting device and the parent endoscope, and when the parent endoscope needs to be disassembled, it is only required that the parent endoscope moves upwards relative to the connecting device, a matching state between the parent endoscope and the connecting device can be released. The connecting device is simple in structure, and the matching between the child endoscope and the parent endoscope are conveniently implemented and released.

### Brief Description of Figures

The accompanying drawings described herein are intended for a further understanding of the present application and constitute a part of the present application. Example embodiments of the present application and descriptions thereof are intended to explain the present application, and do not constitute any inappropriate limitation on the present application. In the figures:
FIG. 1 shows a schematic diagram of a matching structure of a connecting device, a child endoscope, and a parent endoscope provided by an embodiment of the present application;
FIG. 2 shows a schematic diagram of another matching structure of a connecting device, a child endoscope, and a parent endoscope provided by an embodiment of the present application;
FIG. 3 shows a first perspective view (in which a second joint portion is in a separated state) of a first connecting device provided by an embodiment of the present application;
FIG. 4 shows a second perspective view of a first connecting device provided by an embodiment of the present application;
FIG. 5 shows a third perspective view of a first connecting device provided by an embodiment of the present application; and
FIG. 6 shows an exploded view of a second connecting device provided by an embodiment of the present application.

In the figures: 1. connecting device; 11. first joint portion; 111. main housing; 1111. reinforcing rib; 1112. reinforcing back plate; 112. clamping piece; 113. first side edge; 114. second side edge; 12. second joint portion; 121. first opening; 122. second opening; 123. first notch; 13. sheath; 131. sheath main body; 132. port edge covering rib; 133. second notch; 134. notch edge covering rib; 2. child endoscope; 21. functional portion; 22. forceps channel nozzle; 25. rotating wheel; 3. parent endoscope.

### Detailed Description of Embodiments

To make the technical solutions and advantages of the embodiments of the present application clearer, the following further describes the exemplary embodiments of the present application in detail with reference to the accompanying drawings. Apparently, the described embodiments are merely some rather than an exhaustive list of all embodiments of the present application. It should be noted that the embodiments and features in the embodiments of the present application may be combined with each other without conflict.

In the description of the present application and the embodiments thereof, it should be understood that, the orientations or positional relationships indicated by the terms "top", "bottom", "height", etc. are based on those shown in the accompanying drawings, intended only for the convenience of describing the present application and for simplifying the description, and not intended to indicate or imply that the referred device or element must be provided with a particular orientation or constructed and operated with a particular orientation, therefore not allowed to be construed as a limitation of the present application.

In the present application and the embodiments thereof, unless otherwise expressly specified and defined, the terms "arranged", "mounted", "attached", "connected", "fixed", etc. should be understood in a broad sense, for example, a connection may be a fixed connection, a detachable connection, or an integral connection; it may be a mechanical connection, or an electrical connection, or a communication connection; it may be a direct connection or an indirect connection via an intermediate medium; and it may be an internal communication of two elements or an interaction of two elements. For those of ordinary skill in the art, the specific meaning of the forgoing terms in the present application may be understood based on specific situations.

In the present application and the embodiments thereof, unless otherwise expressly specified and defined, a first feature being "above" or "below" a second feature may include not only direct contact between the first and second features but also indirect contact between the first and second features via another feature between them. Furthermore, a first feature being "above", "over" or "at top of" a second feature includes the first feature being directly above and diagonally above the second feature, or simply indicates that the first feature is higher in level than the second feature. A first feature being "below", "beneath" or "under" a second feature includes the first feature being directly below and diagonally below the second feature, or simply indicates that the first feature is lower in level than the second feature.

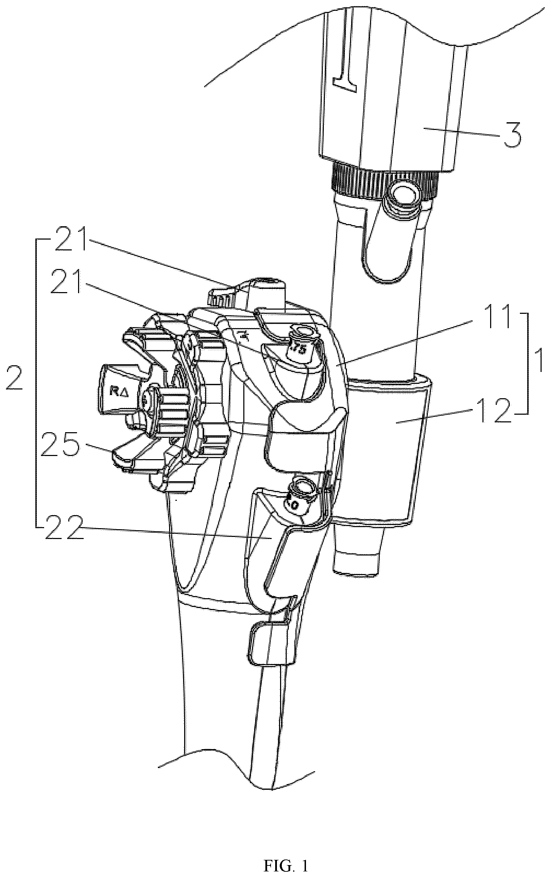

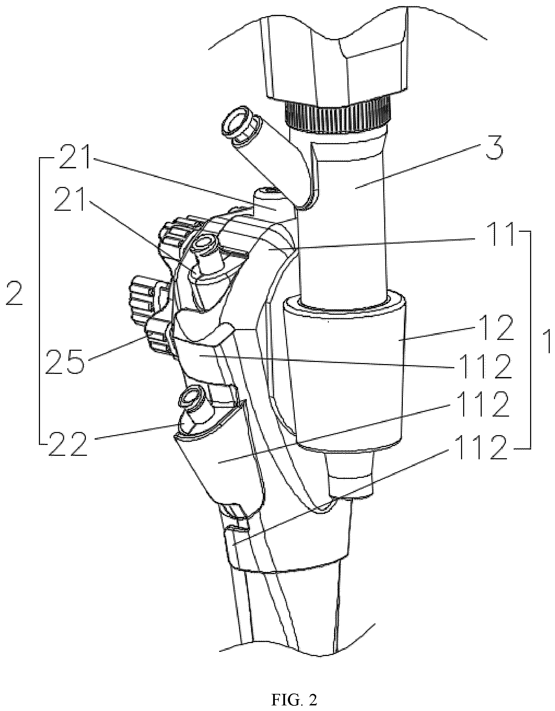

Referring to FIG. 1 to FIG. 6, an embodiment of the present application provides a connecting device 1, which can be used for connecting a child endoscope 2 and a parent endoscope 3, so that the child endoscope and the parent endoscope are fixed at a relative position, and a doctor only needs to hold the child endoscope 2 or the parent endoscope 3, thereby reducing the difficulty in operation of surgery.

In some possible implementation solutions, the connecting device 1 can include a first joint portion 11 and a second joint position 12. The first joint portion 11 is used for connecting the child endoscope 2, the second joint portion 12 is connected to the first joint portion 11, and the second joint portion 12 is detachably connected to the parent endoscope 3.

In the embodiments of the present application, the first joint portion 11 can be detachably connected to the child endoscope 2, and the second joint portion 12 can also be detachably connected to the parent endoscope 3. After the surgery ends, the first joint portion 11 can be disengaged from the child endoscope 2, and the second joint portion 12 can be disengaged from the parent endoscope 3, thereby facilitating cleaning and storing the child endoscope 2 and the parent endoscope 3 separately.

The parent endoscope 3 can be an enteroscope or a gastroscope. Taking the parent endoscope 3 being a duodenoscope as an example, the child endoscope 2 can be a choledochoscope matched with the duodenoscope for use. During the surgery, the doctor first inserts an imaging catheter of the duodenoscope into a natural orifice of a human body and then performs photographing and video recording through the imaging catheter, so that the doctor can observe whether there is diseased tissue (such as polyp or early cancer) in the natural orifice of the human body. If the doctor discovers the diseased tissue, the imaging catheter of the choledochoscope needs to be inserted into a forceps channel hole of the duodenoscope, and then further enters the biliary tract via the imaging catheter of the duodenoscope, so as to perform a calculus removal or in vivo sampling operation. During the surgery, in order to reduce difficulty of the operation, the connecting device 1 can be pre-connected to the choledochoscope through the first joint portion 11. When the choledochoscope is used in a later period, the connecting device 1 can be quickly and effectively matched with the duodenoscope for use through the second joint portion 12, so that the doctor can hold the duodenoscope and the choledochoscope with one hand, thereby facilitating the doctor to operate the choledochoscope for the surgery.

In some possible implementation solutions, referring to FIG. 1 to FIG. 3, the second joint portion 12 encloses to form a sleeving cavity and a first opening 121 and a second opening 122 in communication with the sleeving cavity, a cross-sectional area of the sleeving cavity gradually increases in a direction from the first opening 121 to the second opening 122, and the second joint portion 12 is used for being sleeved on a parent endoscope 3 through the sleeving cavity, allowing the second joint portion 12 to be connected to the parent endoscope 3.

The second joint portion 12 can be of a conical barrel structure, and when the parent endoscope 3 is a gastroscope or an enteroscope, a conical transition section is arranged between a handle and an imaging catheter of the parent endoscope. A big head end of the conical transition section is close to the handle. The sleeving cavity of the second joint portion 12 provided by the embodiment of the present application is a conical cavity. After the second joint portion 12 is sleeved on the imaging catheter of the parent endoscope 3, the second joint portion 12 continues to be moved to one side of the handle of the duodenoscope, so that the second joint portion 12 can be in close contact with the conical transition section of the parent endoscope 3, and the second joint portion 12 can be connected to and matched with the parent endoscope 3. The second joint portion 12 is matched with the conical transition section in an extruded manner, which generates a relatively large frictional resistance and a clamping force, and thus the connecting device 1 cannot be easily disengaged from the conical transition section of the parent endoscope 3.

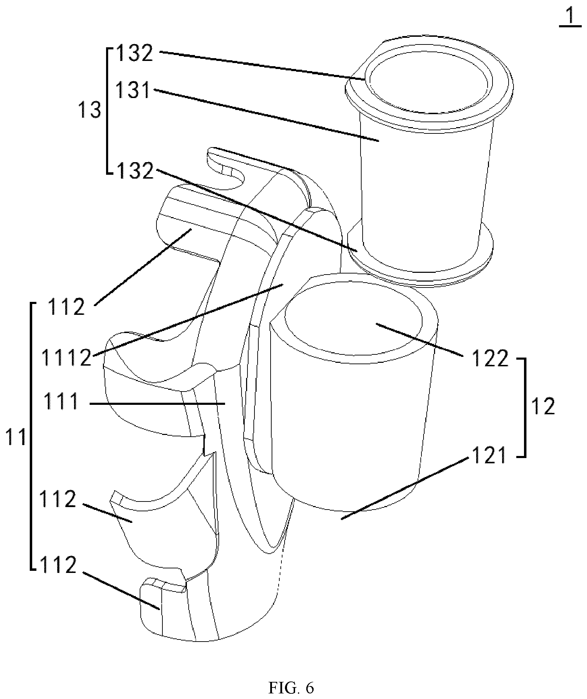

The second joint portion 12 can be arranged in any one of the following two manners:
in a first arrangement manner, referring to FIG. 6, the second joint portion 12 is in a closed ring shape, and an outer wall of the second joint portion 12 is connected to the first joint portion 11. The second joint portion 12 can be a conical cylinder, and the second joint portion 12 needs to be sleeved and fixed on a conical transition section of the parent endoscope 3 in advance before surgery. When the child endoscope 2 needs to be used, the doctor then assembles the child endoscope 2 to the first joint portion 11.

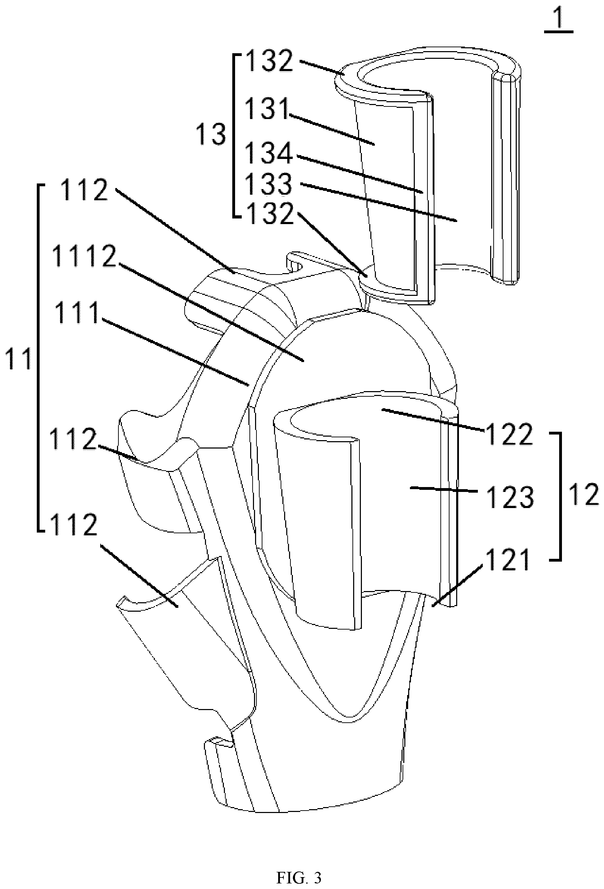

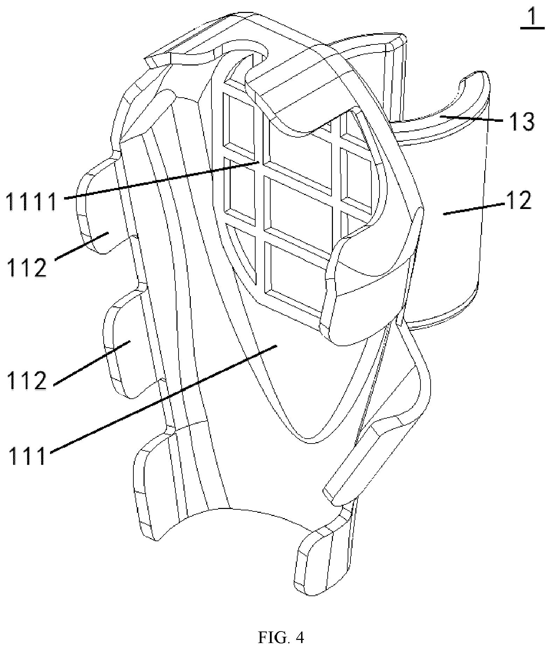

In a second arrangement manner, referring to FIG. 3 and FIG. 4, the second joint portion 12 is provided with a first notch 123, the first notch 123 is in communication with the sleeving cavity, the first notch 123 extends from a first port to a second port, and one side, opposite to the first notch 123, of the second joint portion 12 is connected to the first joint portion 11.

In this implementation solution, the second joint portion 12 is not a closed cylinder but a conical barrel having a long strip-shaped first notch 123. The first notch 123 can be a long strip-shaped opening, and a width of the first notch 123 is not less than an outer diameter of a local structure (an imaging catheter) of the parent endoscope 3, so that the parent endoscope 3 can be extended into the sleeving cavity through the long strip-shaped opening. In the second arrangement manner, the connecting device 1 does not need to be pre-assembled on the parent endoscope 3, instead, the connecting device 1 can be assembled on and connected to the child endoscope 2 through the first joint portion 11, and when the child endoscope 2 needs to be used during the surgery, the second joint portion 12 is assembled on and connected to the parent endoscope 3. During a specific operation, the connecting device 1 can be moved, so that the imaging catheter of the parent endoscope 3 passes through the first notch 123 to enter the sleeving cavity of the second joint portion 12, and then the connecting device 1 is moved upwards, so that the second joint portion 12 can be tightly sleeved on the conical transition section of the parent endoscope 3. When a matching use state needs to be released, the parent endoscope 3 can be moved upwards relative to the connecting device 1, so that the parent endoscope 3 can be quickly separated from the connecting device 1.

In some possible implementation solutions, referring to FIG. 3, the connecting device 1 can include a sheath 13, where the sheath 13 is a flexible body, and the sheath 13 is at least partially located in the sleeving cavity. In a state in which the second joint portion 12 is connected to the parent endoscope 3, the sheath 13 is disposed between the parent endoscope 3 and an inner wall of the sleeving cavity in a compressed manner.

The sheath 13 can be made of a silica gel or rubber material. In a state in which the conical transition section of the parent endoscope 3 penetrates through the second joint portion 12, one surface of the sheath 13 in a thickness direction is attached to a surface of the conical transition section, and the other surface of the sheath 13 in the thickness direction is attached to the inner wall of the sleeving cavity, and thus the sheath 13 plays a role in cushioning protection.

In some possible implementation solutions, referring to FIG. 3, the sheath 13 is provided with a sheath main body 131 and a port edge covering rib 132, the sheath main body 131 is provided with two ports, and the two ports are provided with the port edge covering rib 132, respectively. In a state in which the sheath 13 is connected to the second joint portion 12, the sheath main body 131 is attached to the inner wall of the sleeving cavity, and the two port edge covering ribs 132 are covered on an end surface of an edge of the first opening 121 of the second joint portion 12 and an end surface of an edge of the second opening 122 of the second joint portion 12, respectively.

In this implementation solution, the port edge covering ribs 132 protrude from an outer surface of the sheath main body 131. In a state in which the sheath 13 is connected to the second joint portion 12, the sheath main body 131 is located on an inner side of the sleeving cavity. The port edge covering ribs 132 at two ends of the sheath main body 131 are directly covered on end surfaces of the ports of the second joint portion 12, avoiding that a sharp edge on the end surfaces is in direct contact with the parent endoscope 3 to cut or damage the parent endoscope 3.

It should be noted that, referring to FIG. 6, when the second joint portion 12 is of a closed barrel structure, the sheath 13 can be correspondingly configured as a closed annular cylinder structure. Referring to FIG. 3, when the second joint portion 12 is of a cylinder structure with an opening, the sheath 13 can be correspondingly configured as a barrel structure with an opening.

In some possible implementation solutions, referring to FIG. 3, a second notch 133 is arranged on the sheath main body 131, where the second notch 133 is in communication with the two ports, respectively, the sheath 13 is provided with a notch edge covering rib 134 arranged on the sheath main body 131, and the notch edge covering rib 134 is located at an edge of the second notch 133. In the state in which the sheath 13 is connected to the second joint portion 12, the notch edge covering rib 134 is covered on a thickness end surface of an edge of the first notch 123 of the second joint portion 12.

In this implementation solution, the sheath 13 is not of a closed barrel structure. In the state in which the sheath 13 is connected to the second joint portion 12, the second notch 133 is positioned opposite to the first notch 123, and the second notch 133 is in communication with the first notch 123. The notch edge covering ribs 134 at edges of two sides of the second notch 133 protrude from an outer surface of the sheath main body 131, and the thickness end surface on the first notch 123 of the sheath main body 131 can be smoothly covered.

In some possible implementation solutions, the first joint portion 11 includes a main housing 111 and a plurality of clamping pieces 112, and the second joint portion 12 is connected to the main housing 111. Each clamping piece 112 is connected to the main housing 111, and each clamping piece 112 is successively arranged at intervals along a periphery of an edge of the main housing 111. In a state in which the first joint portion 11 is connected to the child endoscope 2, the main housing 111 is attached to the child endoscope 2, and each clamping piece 112 is clamped to an outer wall surface of a medical instrument, respectively.

Each clamping piece 112 of the first joint portion 11 encloses to form a clamping space. The child endoscope 2 generally includes a handle, the shape of the handle is matched with that of the clamping space, and an outer contour of the handle is slightly larger than the clamping space. In a process of mounting the handle of the child endoscope 2 in the clamping space, the handle of the child endoscope 2 can expand a plurality of clamping pieces 112, and each clamping piece 112 is deformed; and after the handle completely enters the clamping space, the deformation of each clamping piece 112 is recovered, and the clamping pieces are clamped on the handle, so that the handle is connected and fixed to the first joint portion 11. Each clamping piece 112 is elastically attached to different positions of the handle, respectively, so that the connecting device 1 is firmly clamped to the child endoscope 2.

In some possible implementation solutions, referring to FIG. 4, a reinforcing rib 1111 is arranged on a surface of the main housing 111 away from the second joint portion 12. The reinforcing rib 1111 may include a plurality of ribs staggered with each other, to form a mesh-shaped reinforcing rib 1111, thereby enhancing the structural strength of the connecting between a second joint portion and each clamping piece 112, and improving the overall rigidity of the connecting device 1 during use.

In some possible implementation solutions, referring to FIG. 3, a reinforcing back plate 1112 is arranged on a surface of the main housing 111 away from each clamping piece 112, and the second joint portion 12 is connected to the reinforcing back plate 1112. Since the reinforcing back plate 1112 is arranged, the structural strength and rigidity of the main housing 111 are increased, the overall rigidity of the connecting device 1 during use is improved, and the relative movement of the child endoscope 2 and the parent endoscope 3 is reduced as much as possible, so that the child endoscope and the parent endoscope form an integrated structure after being matched and connected with each other through the connecting device 1.

In some possible implementation solutions, the child endoscope 2 is provided with a plurality of functional portions 21. In a state in which the first joint portion 11 is connected to the child endoscope 2, at least part of the functional portions 21 are located on a gap formed between adjacent clamping pieces 112.

When the child endoscope 2 is a choledochoscope, the functional portions 21 can include a photographing key, a locking control key, a liquid injection port, and the like. Positions of the clamping pieces 112 can be arranged to avoid the functional portions 21, without affecting the normal use of the child endoscope 2.

In some possible implementation solutions, the child endoscope 2 is provided with a forceps channel nozzle 22 protruding outwards, and the forceps channel nozzle 22 is provided with a forceps channel opening. In the state in which the first joint portion 11 is connected to the child endoscope 2, one clamping piece 112 is clamped to an outer wall surface of the forceps channel nozzle 22. The handle of the child endoscope 2 is provided with a photographing key, a locking control key, and a liquid injection port, and the handle is further provided with a forceps channel opening. The forceps channel opening is in communication with a forceps channel of the child endoscope 2. The forceps channel nozzle 22 protrudes from the handle and extends a relatively long distance along a smooth peripheral side surface of the handle. If the clamping pieces 112 are staggered with the forceps channel nozzle 22, the problems that a connection structure between the first joint portion 11 and the child endoscope 2 is unstable and clamping release is easy to occur. In the embodiments of the present application, one clamping piece 112 of the clamping pieces 112 can be tightly attached to an outer wall surface of the forceps channel nozzle 22 and does not block the forceps channel opening of the forceps channel nozzle 22, so that each clamping piece 112 on the first joint portion 11 is evenly clamped on the handle of the child endoscope 2, thereby improving the stability of an assembly structure.

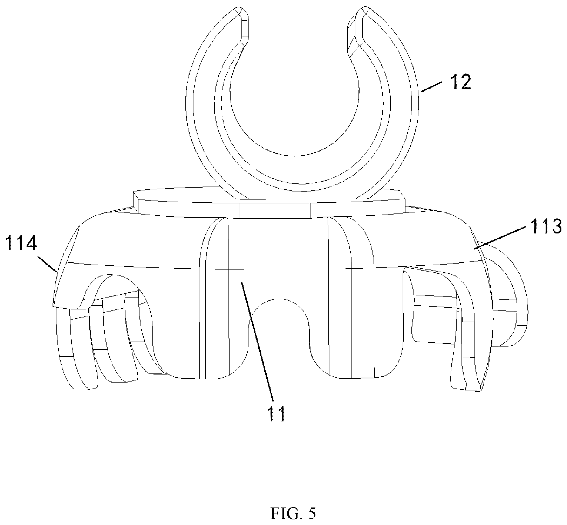

In some possible implementation solutions, referring to FIG. 5, two sides of the first joint portion 11 in a width direction are provided with a first side edge 113 and a second side edge 114, respectively, and a distance between the second joint portion 12 and the first side edge 113 is less than a distance between the second joint portion 12 and the second side edge 114.

When the child endoscope 2 is a choledochoscope, a handle of the child endoscope is provided with a rotating wheel 25, and the rotating wheel 25 is located on one side, away from the first joint portion 11, of the handle. A bending angle and a bending direction of an end portion of an imaging catheter of the child endoscope 2 can be adjusted by rotating the rotating wheel 25. In the embodiments of the present application, the second joint portion 12 is not arranged at a middle position of the first joint portion 11 in a width direction, but is close to a width edge of the first joint portion 11, and the second joint portion 12 is offset out of center, so as to form a lever structure. There is a certain distance between the second joint portion 12 and a rotation axis of the rotating wheel 25, so that torque is increased, and torsion of the rotating wheel 25 of a matched medical instrument in a rotating process can be easily offset, so that when the rotating wheel 25 is rotated, the child endoscope 2 is not overstressed, there will be no large deviation between the handle of the child endoscope 2 and the parent endoscope 3, and the operation accuracy of surgery is improved.

In addition, the embodiments of the present application further provide an endoscope connecting system, including: the above connecting device 1 and a child endoscope 2, where the child endoscope 2 is detachably connected to a first joint portion 11 of the connecting device 1.

The endoscope connecting system further includes a parent endoscope 3, where the parent endoscope 3 is detachably connected to a second joint portion 12 of the connecting device 1.

In the embodiments of the present application, the first joint portion 11 of the connecting device 1 of the endoscope connecting system can be detachably connected to the child endoscope 2, and the second joint portion 12 can also be detachably connected to the parent endoscope 3. After the surgery ends, the first joint portion 11 can be disengaged from the child endoscope 2, and the second joint portion 12 can be disengaged from the parent endoscope 3, thereby facilitating cleaning and storing the child endoscope 2 and the parent endoscope 3 separately.

The second joint portion 12 of the connecting device 1 can be sleeved on the parent endoscope 3, so as to implement a quick matching use of the connecting device 1 and the parent endoscope 3, and when the parent endoscope 3 needs to be disassembled, it is only required that the parent endoscope 3 moves upwards relative to the connecting device 1, so that the parent endoscope 3 and the connecting device 1 can be relatively fixed in a radial direction of an imaging catheter. The connecting device 1 is simple in structure, and the matching between the child endoscope and the parent endoscope are conveniently implemented and released. The first joint portion 11 of the connecting device 1 is matched with the child endoscope 2 in a clamping manner through the plurality of clamping pieces 112.

In some possible implementation solutions, a disassembly direction of the child endoscope 2 and the first joint portion 11 is different from a disassembly direction of the parent endoscope 3 and the second joint portion 12. When an external force is applied to the parent endoscope 3 to release a connection relationship between the parent endoscope 3 and the connecting device 1, a joint relationship between the connecting device 1 and the child endoscope 2 is not affected, and a connection joint state between the connecting device 1 and the child endoscope 2 can be maintained without being affected.

In some possible implementation solutions, an acting force is applied in a horizontal direction of an operating handle of the child endoscope 2, allowing that the child endoscope 2 is split from the first joint portion 11. The term "in a horizontal direction of an operating handle of the child endoscope" can be understood as being substantially in a thickness direction of the operating handle, or can be understood as an arrangement direction of the first joint portion 11 and the second joint portion 12 of the connecting device 1.

An acting force is applied in a vertical direction of an imaging catheter of the parent endoscope 3, allowing that the parent endoscope 3 is split from the second joint portion 12.

It can be seen that the disassembly direction of the child endoscope 2 and the first joint portion 11 can be substantially perpendicular to the disassembly direction of the parent endoscope 3 and the second joint portion 12. When one of the parent endoscope 3 and the child endoscope 2 is disassembled by an external force, a joint connection state of the other one and the connecting device 1 is not affected.

The above disclosure provides many different implementations or examples to implement different structures of the present application. In order to simplify the disclosure of the present application, components and settings of specific examples are described above. Of course, they are merely examples, and are not intended to limit the present application. In addition, reference numerals and/or reference letters can be repeated in different examples in the present application for the purpose of simplicity and clarity, which does not indicate the relationship between the various embodiments and/or settings discussed. In addition, the present application provides examples of various specific processes and materials, but those of ordinary skill in the art may be aware of the application of other processes and/or the use of other materials.

Although some preferred embodiments of the present application have been described, those skilled in the art can make changes and modifications to these embodiments once they learn the basic inventive concept. Therefore, the appended claims are intended to be construed as to cover the preferred embodiments and all changes and modifications falling within the scope of the present application.

Obviously, those skilled in the art can make various modifications and variations to the present application without departing from the spirit and scope of the present application. The present application is intended to cover these modifications and variations of the present application provided that they fall within the scope of protection defined by the appended claims and their equivalent technologies.

## Claims

1. A connecting device, **characterized by** comprising:
a first joint portion used for connecting a child endoscope; and
a second joint portion connected to the first joint portion, wherein the second joint portion encloses to form a sleeving cavity and a first opening and a second opening in communication with the sleeving cavity, a cross-sectional area of the sleeving cavity gradually increases in a direction from the first opening to the second opening, and the second joint portion is used for being sleeved on a parent endoscope through the sleeving cavity, allowing the second joint portion to be connected to the parent endoscope.

2. The connecting device according to claim 1, **characterized in that** the second joint portion is arranged in any one of the following manners:
the second joint portion is in a closed ring shape, and an outer wall of the second joint portion is connected to the first joint portion; and
the second joint portion is provided with a first notch, the first notch is in communication with the sleeving cavity, the first notch extends from a first port to a second port, and one side, opposite to the first notch, of the second joint portion is connected to the first joint portion.

3. The connecting device according to claim 2, **characterized in that** the first notch is a long strip-shaped opening; and
a width of the first notch is not less than an outer diameter of a local structure of the parent endoscope, so that the parent endoscope can extend into the sleeving cavity through the long strip-shaped opening.

4. The connecting device according to claim 2, **characterized by** comprising a sheath, wherein the sheath is a flexible body;
the sheath is at least partially located in the sleeving cavity; and
in a state in which the second joint portion is connected to the parent endoscope, the sheath is disposed between the parent endoscope and an inner wall of the sleeving cavity in a compressed manner.

5. The connecting device according to claim 4, **characterized in that** the sheath is provided with a sheath main body and a port edge covering rib;
the sheath main body is provided with two ports, and the two ports are provided with the port edge covering rib, respectively; and
in a state in which the sheath is connected to the second joint portion, the sheath main body is attached to the inner wall of the sleeving cavity, and the two port edge covering ribs are covered on an end surface of an edge of the first opening of the second joint portion and an end surface of an edge of the second opening of the second joint portion, respectively.

6. The connecting device according to claim 5, **characterized in that** a second notch is arranged on the sheath main body, and the second notch is in communication with the two ports, respectively;
the sheath is provided with a notch edge covering rib arranged on the sheath main body, and the notch edge covering rib is located at an edge of the second notch; and
in the state in which the sheath is connected to the second joint portion, the notch edge covering rib is covered on a thickness end surface of an edge of the first notch of the second joint portion.

7. The connecting device according to claim 1, **characterized in that** the first joint portion comprises a main housing and a plurality of clamping pieces;
the second joint portion is connected to the main housing;
each clamping piece is connected to the main housing, and each clamping piece is successively arranged at intervals along a periphery of an edge of the main housing; and in a state in which the first joint portion is connected to the child endoscope, the main housing is attached to the child endoscope, and each clamping piece is clamped to an outer wall surface of a medical instrument, respectively.

8. The connecting device according to claim 7, **characterized in that** a reinforcing rib is arranged on a surface of the main housing away from the second joint portion.

9. The connecting device according to claim 7, **characterized in that** a reinforcing back plate is arranged on a surface of the main housing away from each clamping piece; and the second joint portion is connected to the reinforcing back plate.

10. The connecting device according to claim 7, **characterized in that** the child endoscope is provided with a plurality of functional portions; and
in a state in which the first joint portion is connected to the child endoscope, at least part of the functional portions are located on a gap formed between adjacent clamping pieces.

11. The connecting device according to claim 7, **characterized in that** the child endoscope is provided with a forceps channel nozzle protruding outwards, and the forceps channel nozzle is provided with a forceps channel opening; and
in the state in which the first joint portion is connected to the child endoscope, one clamping piece is clamped to an outer wall surface of the forceps channel nozzle.

12. The connecting device according to any one of claims 1 to 11, **characterized in that** two sides of the first joint portion in a width direction are provided with a first side edge and a second side edge, respectively; and
a distance between the second joint portion and the first side edge is less than a distance between the second joint portion and the second side edge.

13. An endoscope connecting system, **characterized by** comprising:
the connecting device according to any one of claims 1 to 12; and
a child endoscope detachably connected to a first joint portion of the connecting device.

14. The endoscope connecting system according to claim 13, **characterized by** further comprising:
a parent endoscope detachably connected to a second joint portion.

15. The endoscope connecting system according to claim 14, **characterized in that** a disassembly direction of the child endoscope and the first joint portion is different from a disassembly direction of the parent endoscope and the second joint portion.

16. The endoscope connecting system according to claim 15, **characterized in that** an acting force is applied in a horizontal direction of an operating handle of the child endoscope, allowing that the child endoscope is split from the first joint portion.

17. The endoscope connecting system according to claim 16, **characterized in that** an acting force is applied in a vertical direction of an imaging catheter of the parent endoscope, allowing that the parent endoscope is split from the second joint portion.
